# DEMANDE DE BREVET EUROPEEN

(11) **EP 4 556 015 A1**
(43) Date de publication de la demande: **21.05.2025**
(21) Numéro de dépôt: 24214001.0
(22) Date de dépôt: 19.11.2024
(51) Int. Cl.: A61K 36/185, A61K 8/9789, A61K 8/92, A61Q 19/00, A61P 9/00, A61P 17/00, A61P 19/02, A61P 19/10, A61P 25/28, A61P 37/00

(54) **COMPOSITION COMPRENANT DE LA PEAU GRENADE ET DE L'HUILE DE FRAINE DE GRENADE**

(30) Priorité: 20.11.2023 FR 2312747
(71) Demandeur: Laboratoires Pomega, 74160 Archamps (FR)
(72) Inventeur: BOUGUEDOURA, Soumya, 74160 ARCHAMPS (FR)
(74) Mandataire: Tripoz, Inès

(57) **Abrégé**

La présente invention concerne une composition comprenant de la peau du fruit de Punica granatum, sous forme particulaire ou sous forme d'extrait, et de l'huile de graine du fruit de Punica granatum en mélange, en particulier pour ses applications cosmétiques, thérapeutiques et/ou nutraceutiques.

## Description

La présente invention concerne une composition comprenant de la peau du fruit de *Punica granatum,* sous forme particulaire ou sous forme d'extrait, et de l'huile de graine du fruit de *Punica granatum,* en mélange, en particulier pour ses applications cosmétiques, thérapeutiques et/ou nutraceutiques.

Le *Punica granatum* est un arbuste de la famille de Lythracées, à feuilles caduques, pouvant atteindre jusqu'à 8 mètres de hauteur.

Le fruit de *Punica granatum,* plus communément appelé grenade, est un fruit bien connu et utilisé depuis des siècles dans la médecine traditionnelle, en particulier dans les régions du Moyen-Orient et de l'Afrique du Nord.

Au cours de la dernière décennie, les preuves des effets bénéfiques de la consommation de grenade sur la santé se sont multipliées, principalement grâce à l'augmentation du nombre d'études les effets sur l'Homme du jus de grenade. Sont attribués à la grenade et à sa consommation des effets tels que des activités cardioprotectrices, neuroprotectrices, anticancéreuses, antioxydantes, antimicrobiennes et prébiotiques. Toutefois, s'agissant de la santé humaine, la commission de l'EFSA a rejeté toutes les allégations de santé liées à la consommation de grenade en 2010 et 2015 en raison en particulier de la grande variabilité des conclusions des études.

Les bienfaits de la grenade sont principalement attribués à sa teneur élevée en polyphénols.

La peau de la grenade contient un grand nombre de molécules dont des saponines, des stéroïdes, des alcaloïdes, des tanins, des hydrates de carbone, des flavonoïdes, des flavonols, des anthraquinones, des proanthocyanidines, des glycosides, des sucres réducteurs, des phénols, *etc.* Les composés les plus abondants sont la punicalagine et les dérivés de l'acide ellagique qui sont des polyphénols.

Le jus a une composition similaire à celle de la peau, mais les teneurs en ces différents composés sont plus faibles.

L'huile de graine contient plusieurs acides gras saturés et poly-insaturés tels que l'acide punicique (oméga-5), l'acide linoléique, l'acide α-oléostéarique et l'acide catalpique. L'acide gras le plus abondant est l'acide punique, un acide gras polyinsaturé à longue chaîne oméga-5 qui est un isomère positionnel et géométrique de l'acide α-linolénique.

La composition qualitative et quantitative des bioactifs dans le fruit de la grenade peut varier selon les zones de récolte, le niveau de maturation, les parties du fruit (jus, peau ou graines) et le processus de transformation.

La demande WO2006022502 divulgue l'utilisation d'ellagitanins pour le traitement des maladies auto-immunes. Cette demande ne divulgue pas l'utilisation d'une composition comprenant de la peau du fruit de *Punica granatum,* sous forme particulaire ou sous forme d'extrait, et de l'huile de graine du fruit de *Punica granatum* en mélange.

De plus, les ellagitanins sont purifiés et isolés à partir du péricarpe de grenade par un procédé comprenant de nombreuses étapes de traitement par des produits potentiellement toxiques pour le patient traité et/ou pour l'environnement, notamment l'acétone ou le méthanol.

La demande WO2005097106A1 divulgue une composition comprenant de la punicalagine et de l'acide ellagique extraits à partir de peau du fruit de grenade, notamment pour des applications pharmaceutiques, nutraceutiques et cosmétiques.

Cette demande ne divulgue pas l'utilisation d'une composition comprenant de la peau du fruit de *Punica granatum,* sous forme particulaire ou sous forme d'extrait, et de l'huile de graine du fruit de *Punica granatum* en mélange.

De plus, la punicalagine et l'acide ellagique de la composition selon la demande WO2005097106A1 sont purifiés par un procédé complexe sur colonne adsorbante.

La demande WO2011038116 divulgue une composition pharmaceutique comprenant des polyphénols obtenus à partir de grenade pour le traitement des troubles osseux. Cette demande ne divulgue pas l'utilisation d'une composition comprenant de la peau du fruit de *Punica granatum,* sous forme particulaire ou sous forme d'extrait, et de l'huile de graine du fruit de *Punica granatum* en mélange.

Les polyphénols selon la demande WO2011038116 sont en particulier extraits à partir de peau du fruit de grenade ayant subi des étapes de traitements enzymatiques et de purification sur colonne de résine.

La demande WO2007/004229 A2 divulgue l'utilisation d'un extrait d'huile et d'un extrait de peau de grenade pour promouvoir la régénération de la peau. Cette demande ne divulgue pas de ratios particuliers entre la peau et d'huile de graine de grenade permettant d'obtenir un effet de synergie.

La demande WO2011/038116 A2 divulgue l'utilisation d'un extrait de grenade obtenue à partir du fruit entier pour améliorer la densité osseuse. Cette demande ne divulgue pas de ratios particuliers entre la peau et d'huile de graine de grenade permettant d'obtenir un effet de synergie.

La demande FR3031901A1 extrait de fruit de grenade et son utilisation pour prévenir l'apparition et le développement des lésions cutanées induites par l'exposition chronique aux rayonnements solaires de type UVA. Cette demande ne divulgue pas de ratios particuliers entre la peau et d'huile de graine de grenade permettant d'obtenir un effet de synergie.

On connait également de l'art antérieur le produit commercial SONOTAS, MOISTURISING CREAM FOR FACE, BODY AND HANDS, se présentant sous la forme d'une crème hydratante comprenant notamment des extraits de peau et d'huile de graine de grenade. Il ne divulgue pas de ratios particuliers entre la peau et l'huile de grenade permettant d'obtenir un effet de synergie.

On connait également de l'art antérieur le produit commercial WN PHARMACEUTICALS, CHRONIC JOINT PAIN CAPLETS destiné notamment à protéger ou re construire les cartilages articulaires. Ce produit comprend notamment des extraits de grenade. Il ne divulgue pas de ratios particuliers entre la peau et l'huile de grenade permettant d'obtenir un effet de synergie.

L'article JOHANNINGSMEIER, et al. Annu. rev. food sci. Technol, 2011, résume les différents effets de la grenade connus pour la santé. Il ne divulgue pas de ratios particuliers entre la peau et l'huile de grenade permettant d'obtenir un effet de synergie.

L'article CHAVES, et al., Plant Food Hum. Nutr., 2019, a pour objet la comparaison entre les effets des extraits de peau de grenade et de jus de grenade dans le traitement du cancer de la prostate. Il ne divulgue pas de ratios particuliers entre la peau et l'huile de grenade permettant d'obtenir un effet de synergie.

De manière particulièrement surprenante, la demanderesse a mis au point une composition dont le procédé de fabrication et le produit fini sont sans danger pour le patient et pour l'environnement.

La composition mise au point par la demanderesse comprend une association deux ingrédients actifs dérivés de grenade possédant une activité synergique pour le traitement des troubles inflammatoires au niveau de divers systèmes, et/ou ostéoarticulaires, en particulier de l'arthrose.

Elle est de plus particulièrement recommandée pour des applications dans le traitement des troubles osseux, cartilagineux, cardiovasculaires et/ou cognitifs.

La demanderesse a en particulier mis au point une composition dont les ratios particuliers de teneurs massiques d'huile de graine et de peau de grenade confèrent à la composition un effet synergique particulièrement surprenant pour les activités cosmétiques et/ou thérapeutiques citées.

Elle est enfin particulièrement adaptée à une administration chez l'Homme et l'animal, en particulier chez le chien, le chat et/ou le cheval.

La présente invention a pour objet une composition comprenant de la peau du fruit de *Punica granatum,* sous forme particulaire ou sous forme d'extrait, et de l'huile de graine du fruit de *Punica granatum* en mélange.

Au sens de la présente invention, on entend par « fruit de *Punica granatum* » le fruit communément appelé grenade.

Dans un mode de réalisation, le *Punica granatum* est de variété Wonderful, Molar, Bigfull, NFG-102, Emek, Shani et/ou Acco.

Au sens de la présente invention, on entend par « peau du fruit de *Punica granatum* » les couches entourant les arilles.

Dans un mode de réalisation, la peau du fruit de *Punica granatum* est sous forme particulaire.

Dans un mode de réalisation, la peau du fruit de *Punica granatum* est sous forme particulaire avec des particules de taille comprise dans un intervalle allant de 50 µm à 1 mm.

Dans un mode de réalisation, la peau du fruit de *Punica granatum* est sous forme particulaire avec des particules de taille comprise dans un intervalle allant de 80 µm à 850 µm.

Dans un mode de réalisation, la peau du fruit de *Punica granatum* est sous forme particulaire avec des particules de taille comprise dans un intervalle allant de 100 µm à 600 µm.

Dans un mode de réalisation, la peau du fruit de *Punica granatum* est sous forme particulaire avec des particules de taille comprise dans un intervalle allant de 300 µm à 500 µm.

Dans un mode de réalisation, la peau du fruit de *Punica granatum* sous forme particulaire est un broyat de peau du fruit de *Punica granatum.*

Dans un mode de réalisation, la peau du fruit de *Punica granatum* est sous forme d'extrait.

Dans un mode de réalisation, la peau du fruit de *Punica granatum* sous forme d'extrait est un extrait aqueux de peau du fruit de *Punica granatum.*

Dans un mode de réalisation, la peau du fruit de *Punica granatum* sous forme d'extrait est un extrait aqueux de peau du fruit de *Punica granatum* broyée.

Dans un mode de réalisation, la peau du fruit de *Punica granatum* la peau du fruit de *Punica granatum* est lyophilisée.

Dans un mode de réalisation, la peau du fruit de *Punica granatum* sous forme particulaire ou sous forme d'extrait est lyophilisée.

La lyophilisation de la peau de fruit de *Punica granatum* présente l'avantage d'augmenter la teneur en polyphénols par rapport à un séchage classique en éliminant au moins une partie de l'eau liée. De plus, lors du séchage classique au moins une partie des polyphénols se retrouvent dégradés.

Dans un mode de réalisation, la peau du fruit de *Punica granatum* est un broyat de peau du fruit de *Punica granatum* lyophilisée.

Dans un mode de réalisation, la peau du fruit de *Punica granatum* n'a pas subi d'étape d'extraction sur résine adsorbante.

Dans un mode de réalisation, la peau du fruit de *Punica granatum* sous forme particulaire ou sous forme d'extrait est caractérisée en ce qu'elle comprend au moins un polyphénol.

Dans un mode de réalisation, la peau du fruit de *Punica granatum* sous forme particulaire ou sous forme d'extrait est caractérisée en ce qu'elle comprend au moins un polyphénol choisi dans le groupe comprenant la punicalagine et/ou l'acide ellagique.

Dans un mode de réalisation, la peau du fruit de *Punica granatum* sous forme particulaire ou sous forme d'extrait est caractérisée en ce que sa teneur massique en polyphénols est comprise dans un intervalle allant de 1 % à 60 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en polyphénols est comprise dans un intervalle allant de 3 % à 50 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en polyphénols est comprise dans un intervalle allant de 5 % à 40 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en polyphénols est comprise dans un intervalle allant de 20 % à 35 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 1 % à 50 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 5 % à 40 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 15 % à 45 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 20 % à 30 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 0,5 % à 20 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 1 % à 15 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 3 % à 10 % (m/m) par rapport à la masse totale de la peau du fruit de *Punica granatum* sous forme particulaire ou sous forme d'extrait.

Dans un mode de réalisation, la peau du fruit de *Punica granatum* sous forme particulaire ou sous forme d'extrait est caractérisée en ce que sa teneur massique en punicalagine est comprise dans un intervalle allant de 4 % à 5 % (m/m) par rapport à la masse totale de la peau du fruit de *Punica granatum* sous forme particulaire ou sous forme d'extrait.

Au sens de la présente invention, on entend par « graine du fruit de *Punica granatum* » les arilles du fruit de *Punica granatum* débarrassés de la chair et du jus.

Dans un mode de réalisation, l'huile de graine du fruit de *Punica granatum* est obtenue par pressage à froid.

Dans un mode de réalisation, l'huile de graine du fruit de *Punica granatum* est caractérisée en ce qu'elle comprend des acides gras.

Dans un mode de réalisation, l'huile de graine du fruit de *Punica granatum* est caractérisée en ce qu'elle comprend des acides gras choisis dans le groupe comprenant l'acide punicique.

Dans un mode de réalisation, l'huile de graine du fruit de *Punica granatum* est caractérisée en ce que sa teneur massique en acide punicique est comprise dans un intervalle allant de 40 % à 95 % (m/m) par rapport à la masse totale des acides gras de l'huile de graine du fruit de *Punica granatum.*

Dans un mode de réalisation, l'huile de graine du fruit de *Punica granatum* est caractérisée en ce que sa teneur massique en acide punicique est comprise dans un intervalle allant de 50 % à 90 % (m/m) par rapport à la masse totale des acides gras de l'huile de graine du fruit de *Punica granatum.*

Dans un mode de réalisation, l'huile de graine du fruit de *Punica granatum* est caractérisée en ce que sa teneur massique en acide punicique est comprise dans un intervalle allant de 60 % à 85 % (m/m) par rapport à la masse totale des acides gras de l'huile de graine du fruit de *Punica granatum.*

Dans un mode de réalisation, l'huile de graine du fruit de *Punica granatum* est caractérisée en ce que sa teneur massique en acide punicique est comprise dans un intervalle allant de 70% à 80% (m/m) par rapport à la masse totale des acides gras de l'huile de graine du fruit de *Punica granatum.*

Dans un mode de réalisation, l'huile de graine du fruit de *Punica granatum* est caractérisée en ce que sa teneur massique en acide punicique est comprise dans un intervalle allant de 40 % à 95 % (m/m) par rapport à la masse de l'huile de graine du fruit de *Punica granatum.*

Dans un mode de réalisation, l'huile de graine du fruit de *Punica granatum* est caractérisée en ce que sa teneur massique en acide punicique est comprise dans un intervalle allant de 50 % à 90 % (m/m) par rapport à la masse de l'huile de graine du fruit de *Punica granatum.*

Dans un mode de réalisation, l'huile de graine du fruit de *Punica granatum* est caractérisée en ce que sa teneur massique en acide punicique est comprise dans un intervalle allant de 60 % à 85 % (m/m) par rapport à la masse du fruit de *Punica granatum.*

Dans un mode de réalisation, l'huile de graine du fruit de *Punica granatum* est caractérisée en ce que sa teneur massique en acide punicique est comprise dans un intervalle allant de 60% à 75 % (m/m) par rapport à la masse de l'huile de graine du fruit de *Punica granatum.*

Selon la présente invention, la peau du fruit de *Punica granatum,* sous forme particulaire ou sous forme d'extrait, et l'huile de graine du fruit de *Punica granatum* sont en mélange. On désignera ci-après le mélange de peau du fruit de *Punica granatum,* sous forme particulaire ou sous forme d'extrait, et d'huile de graine du fruit de *Punica granatum* alternativement sous la dénomination « le mélange ».

Le mélange consiste donc en la peau du fruit de *Punica granatum,* sous forme particulaire ou sous forme d'extrait, telle que précédemment définie, et l'huile de graine du fruit de *Punica granatum* telle que précédemment définie.

Dans un mode de réalisation l'huile de graine du fruit de *Punica granatum* est adsorbée sur une substance neutre à base de silice comme une silice mésoporeuse, pour obtenir une huile sous forme pulvérulente.

Dans un mode de réalisation, le mélange est sous forme liquide, solide ou semi-solide.

Dans un mode de réalisation, le mélange sous forme liquide est une dispersion, une émulsion et/ou une suspension.

Dans un mode de réalisation, le mélange sous forme liquide est une émulsion.

Dans un mode de réalisation, le mélange sous forme liquide est une émulsion eau dans huile ou une émulsion huile dans eau.

Dans un mode de réalisation, le mélange sous forme solide est une poudre.

Dans un mode de réalisation, le mélange sous forme solide est une poudre de peau de fruit de *Punica granatum* enrobée d'huile de graine de *Punica granatum* sèche.

Dans un mode de réalisation, le mélange sous forme solide est une poudre de peau de fruit de *Punica granatum* imprégnée d'huile de graine de *Punica granatum.*

Dans un mode de réalisation, le mélange sous forme semi-solide est une suspension et/ou une dispersion.

Dans un mode de réalisation, le mélange est caractérisé en ce que sa teneur massique en polyphénols est comprise dans un intervalle allant de 2 % à 50 % (m/m) par rapport à la masse totale du mélange.

Dans un mode de réalisation, le mélange est caractérisé en ce que sa teneur massique en polyphénols est comprise dans un intervalle allant de 10 % à 40 % (m/m) par rapport à la masse totale du mélange.

Dans un mode de réalisation, le mélange est caractérisé en ce que sa teneur massique en polyphénols est comprise dans un intervalle allant de 20 % à 35 % (m/m) par rapport à la masse totale du mélange.

Dans un mode de réalisation, le mélange est caractérisé en ce que sa teneur massique en punicalagine est comprise dans un intervalle allant de 1 % à 70 % (m/m) par rapport à la masse totale du mélange.

Dans un mode de réalisation, le mélange est caractérisé en ce que sa teneur massique en punicalagine est comprise dans un intervalle allant de 5 % à 50 % (m/m) par rapport à la masse totale du mélange.

Dans un mode de réalisation, le mélange est caractérisé en ce que sa teneur massique en punicalagine est comprise dans un intervalle allant de 15 % à 40 % (m/m) par rapport à la masse totale du mélange.

Dans un mode de réalisation, le mélange est caractérisé en ce que sa teneur massique en punicalagine est comprise dans un intervalle allant de 20 % à 35 % (m/m) par rapport à la masse totale du mélange.

Dans un mode de réalisation, le mélange est caractérisé en ce que sa teneur massique en punicalagine est comprise dans un intervalle allant de 25 % à 30 % (m/m) par rapport à la masse totale du mélange.

Dans un mode de réalisation, le mélange est caractérisé en ce que sa teneur massique en acide ellagique est comprise dans un intervalle allant de 0,5 % à 20 % (m/m) par rapport à la masse totale du mélange.

Dans un mode de réalisation, le mélange est caractérisé en ce que sa teneur massique en acide ellagique est comprise dans un intervalle allant de 1 % à 15 % (m/m) par rapport à la masse totale du mélange.

Dans un mode de réalisation, le mélange est caractérisé en ce que sa teneur massique en acide ellagique est comprise dans un intervalle allant de 3 % à 10 % (m/m) par rapport à la masse totale du mélange.

Dans un mode de réalisation, le mélange est caractérisé en ce que sa teneur massique en acide ellagique est comprise dans un intervalle allant de 4 % à 5 % (m/m) par rapport à la masse totale du mélange.

Dans un mode de réalisation, le mélange est caractérisé en ce que sa teneur massique en acide punicique est comprise dans un intervalle allant de 40 % à 95 % (m/m) par rapport à la masse totale des acides gras du mélange.

Dans un mode de réalisation, le mélange est caractérisé en ce que sa teneur massique en acide punicique est comprise dans un intervalle allant de 50 % à 90 % (m/m) par rapport à la masse totale des acides gras du mélange.

Dans un mode de réalisation, le mélange est caractérisé en ce que sa teneur massique en acide punicique est comprise dans un intervalle allant de 60 % à 85 % (m/m) par rapport à la masse totale des acides gras du mélange.

Dans un mode de réalisation, le mélange est caractérisé en ce que sa teneur massique en acide punicique est comprise dans un intervalle allant de 70% à 80% (m/m) par rapport à la masse totale des acides gras du mélange.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 0,001/1 à 1000/1.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 0,01/1 à 100/1.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/1 à 50/1.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 5/1 à 20/1.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 7/1 à 15/1.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 8/1 à 13/1.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 9/1 à 12/1.

Dans un mode de réalisation, la composition selon la présente invention comprend un ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/1 à 1/50.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/5 à 1/20.

Dans un mode de réalisation, le mélange est caractérisé en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/7 à 1/15.

Dans un mode de réalisation, le mélange comprend de 0,01 mg à 1 g de peau du fruit de *Punica granatum,* sous forme particulaire ou sous forme d'extrait.

Dans un mode de réalisation, le mélange comprend de 0,1 mg à 100 mg de peau du fruit de *Punica granatum,* sous forme particulaire ou sous forme d'extrait.

Dans un mode de réalisation, le mélange comprend de 0,5 mg à 50 mg de peau du fruit de *Punica granatum,* sous forme particulaire ou sous forme d'extrait.

Dans un mode de réalisation, le mélange comprend de 1 mg à 30 mg de peau du fruit de *Punica granatum,* sous forme particulaire ou sous forme d'extrait.

Dans un mode de réalisation, le mélange comprend de 0,01 ml à 1000 ml d'extrait aqueux de peau du fruit de *Punica granatum* broyée.

Dans un mode de réalisation, le mélange comprend de 0,1 ml à 100 ml d'extrait aqueux de peau du fruit de *Punica granatum* broyée.

Dans un mode de réalisation, le mélange comprend de 1 ml à 50 ml d'extrait aqueux de peau du fruit de *Punica granatum* broyée.

Dans un mode de réalisation, le mélange comprend de 0,001 mg à 1 g d'huile de graine du fruit de *Punica granatum.*

Dans un mode de réalisation, le mélange comprend de 0,005 mg à 10 mg d'huile de graine du fruit de *Punica granatum.*

Dans un mode de réalisation, le mélange comprend de 0,01 mg à 2 mg d'huile de graine du fruit de *Punica granatum.*

Dans un mode de réalisation, le mélange comprend de 0,001 µl à 1 ml d'huile de graine du fruit de *Punica granatum.*

Dans un mode de réalisation, le mélange comprend de 0,1 µl à 500 µl d'huile de graine du fruit de *Punica granatum.*

Dans un mode de réalisation, le mélange comprend de 10 µl à 400 µl d'huile de graine du fruit de *Punica granatum.*

Dans un mode de réalisation, le mélange comprend de 50 µl à 200 µl d'huile de graine du fruit de *Punica granatum.*

Dans un mode de réalisation, la composition selon la présente invention est une composition cosmétique, nutraceutique et/ou pharmaceutique et/ou un ingrédient alimentaire et/ou un complément alimentaire

Dans un mode de réalisation, la composition selon la présente invention est une composition cosmétique.

Dans un mode de réalisation, la composition selon la présente invention est une composition nutraceutique.

Dans un mode de réalisation, la composition selon la présente invention est une composition pharmaceutique.

Dans un mode de réalisation, la composition selon la présente invention est un ingrédient alimentaire et/ou un complément alimentaire.

Dans un mode de réalisation, la composition selon la présente invention est un actif cosmétique, nutraceutique et/ou pharmaceutique.

Dans un mode de réalisation, la composition selon la présente invention est un actif cosmétique.

Dans un mode de réalisation, la composition selon la présente invention est un actif nutraceutique.

Dans un mode de réalisation, la composition selon la présente invention est un actif pharmaceutique.

Dans un mode de réalisation, la composition est sous forme liquide, solide ou semi-solide.

Dans un mode de réalisation, la composition sous forme liquide est un concentré, une dispersion, des gouttes, une émulsion et/ou une suspension.

Dans un mode de réalisation, la composition sous forme liquide est une émulsion.

Dans un mode de réalisation, la composition sous forme liquide est une émulsion eau dans huile ou une émulsion huile dans eau.

Dans un mode de réalisation, la composition sous forme solide est un bloc, un cachet, une capsule, un film, des granulés, un chewing-gum, un implant, un matériau imprégné, un insert, une pastille, un lyophilisat, un patch, des pellets, un pessaire, des pilules, un plâtre, une poche, une poudre, un bâton, un suppositoire, un comprimé et/ou une poudre de peau de fruit de *Punica granatum* enrobée d'huile de graine de *Punica granatum* sèche.

Dans un mode de réalisation, la composition sous forme solide est une poudre de peau de fruit de *Punica granatum* enrobée d'huile de graine de *Punica granatum* sèche

Dans un mode de réalisation, la composition sous forme semi-solide est une crème, une mousse, un gel, une pommade, une pâte et/ou un cataplasme.

Dans un mode de réalisation, la composition sous forme semi solide est versée dans un dispositif de délivrance de spécialité liquide comportant un réservoir en gélatine mole et un capuchon fermant hermétiquement le réservoir, comportant en outre une languette disposée du côté opposé du capuchon par rapport au réservoir, la languette étant remplie de gélatine mole tel que décrit dans la demande WO2021156557 (A1).

Dans un mode de réalisation, la composition selon la présente invention est caractérisée en ce que sa teneur massique en mélange est comprise dans un intervalle allant de 1 % à 100 % (m/m) par rapport à la masse totale de la composition.

Dans un mode de réalisation, la composition selon la présente invention est caractérisée en ce que sa teneur massique en mélange est comprise dans un intervalle allant de 5 % à 80 % (m/m) par rapport à la masse totale de la composition.

Dans un mode de réalisation, la composition selon la présente invention est caractérisée en ce que sa teneur massique en mélange est comprise dans un intervalle allant de 10 % à 70 % (m/m) par rapport à la masse totale de la composition.

Dans un mode de réalisation, la composition selon la présente invention consiste en le mélange de peau du fruit de *Punica granatum,* sous forme particulaire ou sous forme d'extrait, et d'huile de graine du fruit de *Punica granatum* par rapport à la masse totale de la composition.

Dans un mode de réalisation, la composition selon la présente invention est caractérisée en ce que sa teneur massique en polyphénol est comprise dans un intervalle allant de 1 % à 60 % (m/m) par rapport à la masse totale de la composition selon la présente invention.

Dans un mode de réalisation, la composition selon la présente invention est caractérisée en ce que sa teneur massique en polyphénol est comprise dans un intervalle allant de 2 % à 50 % (m/m) par rapport à la masse totale de la composition selon la présente invention.

Dans un mode de réalisation, la composition selon la présente invention est caractérisée en ce que sa teneur massique en polyphénol est comprise dans un intervalle allant de 10 % à 40 % (m/m) par rapport à la masse totale de la composition selon la présente invention.

Dans un mode de réalisation, la composition selon la présente invention est caractérisée en ce que sa teneur massique en polyphénol est comprise dans un intervalle allant de 20 % à 35 % (m/m) par rapport à la masse totale de la composition selon la présente invention.

Dans un mode de réalisation, la composition selon la présente invention est caractérisée en ce que sa teneur massique en punicalagine est comprise dans un intervalle allant de 1 % à 70 % (m/m) par rapport à la masse totale de la composition selon la présente invention.

Dans un mode de réalisation, la composition selon la présente invention est caractérisée en ce que sa teneur massique en punicalagine est comprise dans un intervalle allant de 5 % à 50 % (m/m) par rapport à la masse totale de la composition selon la présente invention.

Dans un mode de réalisation, la composition selon la présente invention est caractérisée en ce que sa teneur massique en punicalagine est comprise dans un intervalle allant de 15 % à 40 % (m/m) par rapport à la masse totale de la composition selon la présente invention.

Dans un mode de réalisation, la composition selon la présente invention est caractérisée en ce que sa teneur massique en punicalagine est comprise dans un intervalle allant de 20 % à 35 % (m/m) par rapport à la masse totale de la composition selon la présente invention.

Dans un mode de réalisation, la composition selon la présente invention est caractérisée en ce que sa teneur massique en punicalagine est comprise dans un intervalle allant de 25 % à 30 % (m/m) par rapport à la masse totale de la composition selon la présente invention.

Dans un mode de réalisation, la composition selon la présente invention est caractérisée en ce que sa teneur massique en acide ellagique est comprise dans un intervalle allant de 0,5 % à 20 % (m/m) par rapport à la masse totale de la composition selon la présente invention.

Dans un mode de réalisation, la composition selon la présente invention est caractérisée en ce que sa teneur massique en acide ellagique est comprise dans un intervalle allant de 1 % à 15 % (m/m) par rapport à la masse totale de la composition selon la présente invention.

Dans un mode de réalisation, la composition selon la présente invention est caractérisée en ce que sa teneur massique en acide ellagique est comprise dans un intervalle allant de 3 % à 10 % (m/m) par rapport à la masse totale de la composition selon la présente invention.

Dans un mode de réalisation, la composition selon la présente invention est caractérisée en ce que sa teneur massique en acide ellagique est comprise dans un intervalle allant de 4 % à 5 % (m/m) par rapport à la masse totale de la composition selon la présente invention.

Dans un mode de réalisation, la composition selon la présente invention est caractérisée en ce que sa teneur massique en acide punicique est comprise dans un intervalle allant de 40 % à 95 % (m/m) par rapport à la masse totale des acides gras de la composition selon la présente invention.

Dans un mode de réalisation, la composition selon la présente invention est caractérisée en ce que sa teneur massique en acide punicique est comprise dans un intervalle allant de 50 % à 90 % (m/m) par rapport à la masse totale des acides gras de la composition selon la présente invention.

Dans un mode de réalisation, la composition selon la présente invention est caractérisée en ce que sa teneur massique en acide punicique est comprise dans un intervalle allant de 60 % à 85 % (m/m) par rapport à la masse totale des acides gras de la composition selon la présente invention.

Dans un mode de réalisation, la composition selon la présente invention est caractérisée en ce que sa teneur massique en acide punicique est comprise dans un intervalle allant de 70 % à 80 % (m/m) par rapport à la masse totale des acides gras de la composition selon la présente invention.

Dans un mode de réalisation, la composition selon la présente invention ne comprend pas de jus de *Punica granatum.*

Dans un mode de réalisation, la composition selon la présente invention ne comprend pas de chair du fruit de *Punica granatum.*

Dans un mode de réalisation, la composition selon la présente invention ne comprend pas de jus de *Punica granatum* et/ou ne comprend pas de chair du fruit de *Punica granatum.*

Dans un mode de réalisation, la composition selon la présente invention ne comprend pas de jus de *Punica granatum* ni de chair du fruit de *Punica granatum.*

Dans un mode de réalisation, la composition selon la présente invention ne comprend pas d'urolithine ou d'isourolithine, en particulier pas d'urolithine A, d'urolithine B, d'urolithine C, d'urolithine D, d'urolithine E, d'urolithine M3, d'urolithine M4, d'urolithine M5, d'urolithine M6, d'urolithine M7 et d'isourolithine A et/ou d'isourolithine B.

Dans un mode de réalisation, la composition selon la présente invention ne comprend pas de jus de *Punica granatum* et/ou de chair du fruit de *Punica granatum,* d'urolithine ou d'isourolithine, en particulier pas d'urolithine A, d'urolithine B, d'urolithine C, d'urolithine D, d'urolithine E, d'urolithine M3, d'urolithine M4, d'urolithine M5, d'urolithine M6, d'urolithine M7 et d'isourolithine A et/ou d'isourolithine B.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 0,001/1 à 1000/1.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 0,01/1 à 100/1.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/1 à 50/1.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 5/1 à 20/1.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 7/1 à 15/1.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 9/1 à 12/1.

Dans un mode de réalisation, la composition selon la présente invention comprend un ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/1 à 1/50.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/5 à 1/20.

Dans un mode de réalisation, la composition selon l'invention est caractérisée en ce que le ratio entre la teneur massique de peau du fruit de *Punica granatum,* sous forme particulaire ou sous forme d'extrait, et la teneur massique d'huile de graine du fruit de *Punica granatum* est compris dans un intervalle allant de 1/7 à 1/15.

Dans un mode de réalisation, la composition selon l'invention comprend de 0,01 mg à 1 g de peau du fruit de *Punica granatum,* sous forme particulaire ou sous forme d'extrait.

Dans un mode de réalisation, la composition selon l'invention comprend de 0,1 mg à 100 mg de peau du fruit de *Punica granatum,* sous forme particulaire ou sous forme d'extrait.

Dans un mode de réalisation, la composition selon l'invention comprend de 0,5 mg à 50 mg de peau du fruit de *Punica granatum,* sous forme particulaire ou sous forme d'extrait.

Dans un mode de réalisation, la composition selon l'invention comprend de 1 mg à 30 mg de peau du fruit de *Punica granatum,* sous forme particulaire ou sous forme d'extrait.

Dans un mode de réalisation, la composition selon l'invention comprend de 0,001 mg à 1 g d'huile de graine du fruit de *Punica granatum.*

Dans un mode de réalisation, la composition selon l'invention comprend de 0,005 mg à 10 mg d'huile de graine du fruit de *Punica granatum.*

Dans un mode de réalisation, la composition selon l'invention comprend de 0,01 mg à 2 mg d'huile de graine du fruit de *Punica granatum.*

Dans un mode de réalisation, la composition selon la présente invention comprend en outre au moins un excipient pharmaceutiquement et/ou cosmétiquement acceptable.

Dans un mode de réalisation, la composition selon la présente invention comprend en outre au moins un excipient pharmaceutiquement et/ou cosmétiquement acceptable choisi dans la liste présentée dans le Règlement (UE) N° 1129/2011 de la Commission Européenne du 11 novembre 2011.

Dans un mode de réalisation, la composition selon la présente invention comprend en outre au moins un excipient pharmaceutiquement et/ou cosmétiquement acceptable choisi dans la liste présentée dans la Directive 2002/46/CE du Parlement européen et du Conseil du 10 juin 2002.

Dans un mode de réalisation, la composition selon la présente invention comprend en outre au moins un excipient pharmaceutiquement et/ou cosmétiquement acceptable choisi dans le groupe comprenant : les tensioactifs, les polymères, les copolymères, les polymères hydrosolubles, les humectants, les composants gras/huileux, les huiles durcies, les triglycérides et diglycérides synthétiques, les cires, les hydrocarbures, les acides gras supérieurs, les alcools supérieurs, les esters, les huiles essentielles, les huiles de silicone, les sels inorganiques, les acides inorganiques, les colorants, les liants, les désintégrants, les diluants, les agents aromatisants, les charges, les lubrifiants, les agents mouillants, les agents de suspension, les agents émulsifiants, les conservateurs antimicrobien et/ou antioxydant, les édulcorants, les prébiotiques, les probiotiques, le tourteau d'huile de graine de grenade, les agents chélateurs et/ou complexant, les agents d'enrobage, les émollients, les agents de glissement, les plastifiants, les solvants aqueux, les appétents vétérinaires et/ou les sels tampons.

Dans un mode de réalisation, la composition selon la présente invention comprend en outre au moins un agent chélateur et/ou complexant.

Dans un mode de réalisation, la composition selon la présente invention comprend en outre au moins un agent chélateur et/ou complexant choisi dans le groupe comprenant la cyclodextrine, l'acide citrique et/ou l'acide malique.

Dans un mode de réalisation, la composition selon la présente invention comprend en outre au moins un émollient.

Dans un mode de réalisation, la composition selon la présente invention comprend en outre au moins un émollient choisi dans le groupe comprenant les huiles végétales, les huiles minérales, le xylitol et/ou le propylène glycol.

Dans un mode de réalisation, la composition selon la présente invention comprend en outre au moins un agent de glissement.

Dans un mode de réalisation, la composition selon la présente invention comprend en outre au moins un agent de glissement choisi dans le groupe comprenant la silice colloïdale.

Dans un mode de réalisation, la composition selon la présente invention comprend en outre au moins un agent d'enrobages.

Dans un mode de réalisation, la composition selon la présente invention comprend en outre au moins un agent d'enrobages choisi dans le groupe comprenant les dérivés cellulosiques, les polyvinyles, l'amidon.

Dans un mode de réalisation, la composition selon la présente invention comprend en outre au moins un plastifiant.

Dans un mode de réalisation, la composition selon la présente invention comprend en outre au moins un plastifiant choisi dans le groupe comprenant l'acétyltributyl citrate, la glycérine, le mannitol et/ou sorbitol.

Dans un mode de réalisation, la composition selon la présente invention comprend en outre au moins un tensioactif.

Au sens de la présente invention, on entend par tensioactif un composé qui modifie la tension superficielle entre deux surfaces, phases. De manière générale, ces composés sont des molécules amphiphiles et permettent de solubiliser deux phases non miscibles d'une composition.

Dans un mode de réalisation, le tensioactif sont choisis dans le groupe comprenant les tensioactifs anioniques, les tensioactifs cationiques, les tensioactifs non ioniques, et/ou les tensioactifs à base de silicone et/ou les tensioactifs à base de fluor.

Dans un mode de réalisation, les tensioactifs anioniques sont choisis dans le groupe comprenant les savons d'acides gras, les tensioactifs anioniques de type sulfonate, les tensioactifs anioniques de type sulfate, les tensioactifs anioniques de type phosphate, les sels d'acylméthyltaurine, les phosphates de monoalkyle, les acylglutamates, et/ou les sels d'ester d'acide iséthionique.

Dans un mode de réalisation, les tensioactifs cationiques sont choisis dans le groupe comprenant les tensioactifs cationiques de type sel d'amine et/ou les tensioactifs cationiques de type ammonium quaternaire (type tétraalkylammonium et type pyridinium).

Dans un mode de réalisation, les tensioactifs non ioniques sont choisis dans le groupe comprenant les esters d'acides gras de glycérine, les esters d'acides gras de propylène glycol, les esters d'acides gras de sorbitan, les polyoxyéthylène esters d'acides gras de sorbitan, l'acide tétraoléique polyoxyéthylène sorbitol, les éthers alkyliques de polyoxyéthylène, les polyoxyéthylène polyoxypropylène glycols, les polyoxyéthylène les éthers alkyliques de polyoxypropylène, les esters d'acides gras de polyéthylèneglycol, les huiles de ricin polyoxyéthylénées, les huiles de ricin hydrogénées de polyoxyéthylène, et/ou les esters d'acides gras de polyglycérine.

Dans un mode de réalisation, les tensioactifs amphotères sont choisis dans le groupe comprenant les tensioactifs de type imidazoline, de type bétaïne et/ou de type acide aminé.

Dans un mode de réalisation, la composition selon la présente invention comprend en outre au moins un polymère hydrosoluble.

Dans un mode de réalisation, le polymère hydrosoluble est choisi dans le groupe comprenant l'alginate de sodium, les esters de propylène glycol de l'acide alginique, la gomme arabique, la gomme xanthane, la pectine, la gomme adragante, la carboxyméthylcellulose de sodium, la méthylcellulose, les polymères carboxyvinyliques, le polyéthylène glycol, l'alcool polyvinylique, la polyvinylpyrrolidone, la cellulose cationisée, le dextrane cationisé, la dextrine cationisée, le chitosane, les polymères de vinylpyrrolidone cationisés, les polymères de chlorure de N,N-diméthyl-3,5-méthylènepipéridinium, la protéine de lait, la protéine de soja, la gélatine, la protéine d'oeuf, la caséine sodique et/ou la protéine de lactosérum.

Dans un mode de réalisation, la composition selon la présente invention comprend en outre au moins un humectant.

Dans un mode de réalisation, l'humectant est choisi dans le groupe comprenant des polyols, des acides aminés, des mucopolysaccharides, des protéines, des extraits biologiques, des métabolites de fermentation, des polysaccharides, des extraits de plantes, des phospholipides et/ou des céramides.

Dans un mode de réalisation, la composition selon la présente invention comprend en outre au moins un émollient.

Dans un mode de réalisation, l'émollient est choisi dans le groupe comprenant des graisses et huiles, notamment l'huile de soja, l'huile de son, l'huile de jojoba, l'huile d'avocat, l'huile d'amande, l'huile de cacao, l'huile d'olive, l'huile de sésame, l'huile persique, l'huile de ricin et/ou l'huile de palme, l'huile de maïs, les huiles minérales, l'huile de vison, le suif de boeuf et/ou le saindoux.

Dans un mode de réalisation, la composition selon la présente invention comprend en outre au moins un triglycéride et/ ou un diglycéride synthétique.

Dans un mode de réalisation, les triglycérides et diglycérides synthétiques sont choisis dans le groupe comprenant le glycéride d'acide myristique et/ou le glycéride d'acide 2-éthylhexanoïque.

Dans un mode de réalisation, la composition selon la présente invention comprend en outre au moins une cire.

Dans un mode de réalisation, la cire est choisie dans le groupe comprenant la cire de carnauba, le spermaceti, la cire d'abeille et/ou la lanoline.

Dans un mode de réalisation, la composition selon la présente invention comprend en outre au moins un hydrocarbure.

Dans un mode de réalisation, l'hydrocarbure est choisi dans le groupe comprenant la paraffine, la vaseline, la cire microcristalline, la cérésine, le squalane et/ou le pristane.

Dans un mode de réalisation, la composition selon la présente invention comprend en outre au moins un acide gras supérieur.

Dans un mode de réalisation, l'acide gras supérieur est choisi dans le groupe comprenant l'acide laurique, l'acide myristique, l'acide palmitique, l'acide stéarique, l'acide béhénique, l'acide oléique, l'acide linoléique et/ou l'acide isostéarique.

Dans un mode de réalisation, les alcools supérieurs sont choisis dans le groupe comprenant l'alcool laurique, l'alcool cétylique, l'alcool stéarylique, l'alcool oléique, le cholestérol et/ou le 2-hexyldécanol.

Dans un mode de réalisation, la composition selon la présente invention comprend en outre au moins un ester.

Dans un mode de réalisation, l'ester est choisi dans le groupe comprenant l'octanoate de cétyle, le lactate de myristyle, le lactate de cétyle, le myristate d'isopropyle, le myristate de myristyle, le palmitate d'isopropyle, l'adipate d'isopropyle, le stéarate de butyle, l'oléate de décyle et/ou l'isostéarate de cholestérol.

Dans un mode de réalisation, la composition selon la présente invention comprend en outre au moins une huile essentielle.

Dans un mode de réalisation, l'huile essentielle est choisie dans le groupe comprenant l'huile de mentha, l'huile de jasmin, l'huile de camphre, l'huile de cyprès japonais, l'huile d'orange amère, l'huile de ryu, l'huile de térébenthine, l'huile de cannelle, l'huile de bergamote, l'huile de mandarine, l'huile de calamus, l'huile de pin, l'huile de lavande, l'huile de laurier, l'huile de clou de girofle, l'huile de hiba, l'huile de rose, l'huile d'eucalyptus, l'huile de citron, l'huile de menthe poivrée, l'huile de thym, l'huile de rose, l'huile de sauge, le menthol, le cinéole, l'eugénol, le citral, le citronellal, le bornéol, le linalol, le géraniol, le camphre, le thymol, le spilanthol, le pinène, le limonène et/ou les composés terpénoïdes.

Dans un mode de réalisation, la composition selon la présente invention comprend en outre au moins un sel inorganique.

Dans un mode de réalisation, les sels inorganiques sont choisis dans le groupe comprenant le carbonate de sodium, l'hydrogénocarbonate de sodium, le sesquicarbonate de sodium, le borax, le sulfate de sodium, le sulfure de sodium, le nitrate de sodium, le thiosulfate de sodium, le polyphosphate de sodium, le phosphate de sodium, le chlorure de potassium, le sulfure de potassium, l'oxyde de calcium, l'oxyde de magnésium, le carbonate de calcium et/ou le carbonate de magnésium.

Dans un mode de réalisation, la composition selon la présente invention comprend en outre au moins un acide inorganique.

Dans un mode de réalisation, les acides inorganiques sont choisis dans le groupe comprenant l'acide borique, l'acide métasilicique et/ou l'anhydride silicique.

Dans un mode de réalisation, les colorants sont choisis dans le groupe comprenant le jaune n° 4, le bleu n° 1, le jaune n° 202, la chlorophylle, la riboflavine, le carthame, la crocine et/ou l'anthraquinone.

Dans un mode de réalisation, la composition selon la présente invention comprend en outre au moins un polymère.

Dans un mode de réalisation, les polymères sont choisis dans le groupe comprenant des résines acryliques, des résines de styrène, des résines époxy, du nylon, du polyéthylène, du polypropylène, du polychlorure de vinyle, des résines de polyéthylène téréphtalate et du polytétrafluoroéthane, et/ou les copolymères de ces polymères.

Dans un mode de réalisation, la composition selon la présente invention comprend en outre au moins un liant.

Dans un mode de réalisation, les liants sont choisis dans le groupe comprenant la cellulose cristalline, les dérivés de cellulose, la gomme arabique, l'amidon de maïs, l'amidon prégélatinisé, la polyvinylpyrrolidone, l'hydroxypropylméthylcellulose ou les gélatines.

Dans un mode de réalisation, la composition selon la présente invention comprend en outre au moins un désintégrant.

Dans un mode de réalisation, les désintégrants sont choisis dans le groupe comprenant l'amidon de maïs, la fécule de pomme de terre, la carboxyméthylcellulose de sodium, l'acide alginique, le L-HPC et/ou le glycolate d'amidon sodique.

Dans un mode de réalisation, la composition selon la présente invention comprend en outre au moins une charge.

Dans un mode de réalisation, les charges sont choisies dans le groupe comprenant les glucides, en particulier le lactose, la cellulose microcristalline et/ou l'hydrogénophosphate de calcium.

Dans un mode de réalisation, la composition selon la présente invention comprend en outre au moins un lubrifiant.

Dans un mode de réalisation, les lubrifiants sont choisis dans le groupe comprenant le stéarate de magnésium, le talc et/ou la silice.

Dans un mode de réalisation, la composition selon la présente invention comprend en outre au moins un lubrifiant.

Dans un mode de réalisation, les agents mouillants sont choisis dans le groupe comprenant le laurylsulfate de sodium.

Dans un mode de réalisation, la composition selon la présente invention comprend en outre au moins un agent de suspension.

Dans un mode de réalisation, les agents de suspension sont choisis dans le groupe comprenant le sirop de sorbitol, des dérivés de cellulose, la gélatine animale et/ou végétale, l'agar, la gomme guar, la gomme arabique, la gomme xanthane, la pectine, le carregeenan, le sirop de mannitol et/ou des graisses comestibles hydrogénées.

Dans un mode de réalisation, la composition selon la présente invention comprend en outre au moins un agent émulsifiant.

Dans un mode de réalisation, les agents émulsifiants sont choisis dans le groupe comprenant la lécithine et/ou l'acacia.

Dans un mode de réalisation, la composition selon la présente invention comprend en outre au moins un agent conservateur.

Dans un mode de réalisation, les conservateurs sont choisis dans le groupe comprenant les p-hydroxybenzoates de méthyle ou de propyle, l'acide ascorbique, l'acide citrique, l'acide malique, la vitamine E et/ou l'acide sorbique.

Dans un mode de réalisation, la composition selon la présente invention comprend en outre au moins un appétent vétérinaire.

Dans un mode de réalisation, la composition selon la présente invention comprend en outre au moins un appétent vétérinaire choisi dans le groupe comprenant les levures, le foie de porc et/ou le foie de volaille.

Dans un mode de réalisation, la composition selon la présente invention comprend en outre au moins un appétent vétérinaire choisi dans le groupe comprenant les levures, en particulier sous forme de paillette.

Dans un mode de réalisation, la composition selon la présente invention comprend en outre au moins un actif pharmaceutique, notamment vétérinaire.

Dans un mode de réalisation, la composition selon la présente invention comprend en outre au moins un actif pharmaceutique pour le traitement et/ou la prévention des troubles articulaires, osseux, cartilagineux, inflammatoires, cardiovasculaires et/ou cognitifs.

Dans un mode de réalisation, la composition selon la présente invention comprend en outre des tourteaux d'huile de graine de *Punica granatum,* de la vitamine A, D et/ou E et/ou des extraits de levure.

Dans un mode de réalisation, la composition selon la présente invention comprend en outre des tourteaux d'huile de graine de *Punica granatum.*

Dans un mode de réalisation, la composition selon la présente invention comprend en outre au moins un actif pharmaceutique pour le traitement et/ou la prévention de la dermatite atopique, l'arthrose, de la polyarthrite rhumatoïde et/ou de l'ostéoporose et/ou pour maintenir et/ou améliorer le remodelage osseux.

Dans un mode de réalisation, la composition selon la présente invention comprend en outre au moins un actif cosmétique.

L'invention concerne également l'utilisation cosmétique de la composition selon la présente invention pour maintenir et/ou améliorer la brillance, l'hydratation et/ou la résistance des phanères.

Dans un mode de réalisation, les phanères sont choisis dans le groupe comprenant les poils et/ou les cheveux.

Dans un mode de réalisation, les phanères sont choisis dans le groupe comprenant les poils.

L'invention concerne également la composition selon la présente invention pour son utilisation thérapeutique et/ou nutraceutique.

Au sens de la présente invention, on entend par « nutraceutique » l'utilisation d'un aliment ou d'un complément alimentaire pour prévenir et/ou traiter les maladies humaines et/ou animales, en particulier pour restaurer, corriger et/ou modifier leurs fonctions physiologiques en exerçant une action pharmacologique, immunologique ou métabolique.

Un mode de réalisation a également pour objet la composition selon la présente invention pour son utilisation thérapeutique et/ou nutraceutique selon la présente invention dans laquelle la composition est administrée chez le mammifère.

Un mode de réalisation a également pour objet la composition selon la présente invention pour son utilisation thérapeutique et/ou nutraceutique selon la présente invention dans laquelle la composition est administrée chez l'Homme.

Un mode de réalisation a également pour objet la composition selon la présente invention pour son utilisation thérapeutique et/ou nutraceutique selon la présente invention dans laquelle la composition est administrée chez l'animal.

Un mode de réalisation a également pour objet la composition selon la présente invention pour son utilisation thérapeutique et/ou nutraceutique selon la présente invention dans laquelle la composition est administrée chez l'animal choisi dans le groupe comprenant le chien, le chat et/ou le cheval.

Un mode de réalisation a également pour objet la composition selon la présente invention pour son utilisation thérapeutique et/ou nutraceutique selon la présente invention dans laquelle la composition est administrée chez l'animal choisi dans le groupe comprenant le chien et/ou le cheval.

Un mode de réalisation a également pour objet la composition selon la présente invention pour son utilisation thérapeutique et/ou nutraceutique selon la présente invention dans laquelle la composition est administrée chez le chien.

Un mode de réalisation a également pour objet la composition selon la présente invention pour son utilisation thérapeutique et/ou nutraceutique selon la présente invention dans laquelle la composition est administrée chez le chat.

Un mode de réalisation a également pour objet la composition selon la présente invention pour son utilisation thérapeutique et/ou nutraceutique selon la présente invention dans laquelle la composition est administrée chez le cheval.

Un mode de réalisation a également pour objet la composition selon la présente invention pour son utilisation thérapeutique et/ou nutraceutique selon la présente invention pour le traitement et/ou la prévention des troubles articulaires, osseux, des cartilagineux, inflammatoires, cardiovasculaires et/ou cognitifs.

Un mode de réalisation a également pour objet la composition selon la présente invention pour son utilisation thérapeutique et/ou nutraceutique selon la présente invention pour le traitement et/ou la prévention de la dermatite atopique, de l'arthrose, de la polyarthrite rhumatoïde et/ou de l'ostéoporose et/ou pour maintenir et/ou améliorer le remodelage osseux.

Un mode de réalisation a également pour objet la composition selon la présente invention pour son utilisation thérapeutique et/ou nutraceutique selon la présente invention dans laquelle elle est administrée par voie orale, parentérale, topique, rectale, auriculaire, nasale et/ou ophtalmique.

Un mode de réalisation a également pour objet la composition selon la présente invention pour son utilisation thérapeutique et/ou nutraceutique selon la présente invention dans laquelle elle est administrée par voie orale.

Un mode de réalisation a également pour objet la composition selon la présente invention pour son utilisation thérapeutique et/ou nutraceutique selon la présente invention dans laquelle la composition est administrée en une quantité permettant l'administration d'une dose de peau du fruit de *Punica granatum* par unité de poids de corps, sous forme particulaire ou sous forme d'extrait, comprise dans un intervalle allant de 50 µg /kg à 100 mg /kg.

Un mode de réalisation a également pour objet la composition selon la présente invention pour son utilisation thérapeutique et/ou nutraceutique selon la présente invention dans laquelle la composition est administrée en une quantité permettant l'administration d'une dose de peau du fruit de *Punica granatum* par unité de poids de corps, sous forme particulaire ou sous forme d'extrait, comprise dans un intervalle allant de 100 µg /kg à 10 mg /kg.

Un mode de réalisation a également pour objet la composition selon la présente invention pour son utilisation thérapeutique et/ou nutraceutique selon la présente invention dans laquelle la composition est administrée en une quantité permettant l'administration d'une dose de peau du fruit de *Punica granatum* par unité de poids de corps, sous forme particulaire ou sous forme d'extrait, comprise dans un intervalle allant de 500 µg /kg à 1 mg /kg.

Un mode de réalisation a également pour objet la composition selon la présente invention pour son utilisation thérapeutique et/ou nutraceutique selon la présente invention dans laquelle la composition est administrée en une quantité permettant l'administration d'une dose de peau du fruit de *Punica granatum* par unité de poids de corps, sous forme particulaire ou sous forme d'extrait, comprise dans un intervalle allant de 800 µg /kg à 900 µg /kg.

Un mode de réalisation a également pour objet la composition selon la présente invention pour son utilisation thérapeutique et/ou nutraceutique selon la présente invention dans laquelle la composition est administrée en une quantité permettant l'administration d'une dose de peau du fruit de *Punica granatum* par unité de poids de corps, sous forme particulaire ou sous forme d'extrait, de 863 µg /kg.

Un mode de réalisation a également pour objet la composition selon la présente invention pour son utilisation thérapeutique et/ou nutraceutique selon la présente invention dans laquelle la composition est administrée en une quantité permettant l'administration d'une dose d'huile de graine du fruit de *Punica granatum* par unité de poids de corps comprise dans un intervalle allant de 1 µg /kg à 1 mg /kg.

Un mode de réalisation a également pour objet la composition selon la présente invention pour son utilisation thérapeutique et/ou nutraceutique selon la présente invention dans laquelle la composition est administrée en une quantité permettant l'administration d'une dose d'huile de graine du fruit de *Punica granatum* comprise dans un intervalle allant de 10 µg/kg à 500 µg/kg.

Un mode de réalisation a également pour objet la composition selon la présente invention pour son utilisation thérapeutique et/ou nutraceutique selon la présente invention dans laquelle la composition est administrée en une quantité permettant l'administration d'une dose d'huile de graine du fruit de *Punica granatum* par unité de poids de corps comprise dans un intervalle allant de 50 µg/kg à 100 µg/kg.

Un mode de réalisation a également pour objet la composition selon la présente invention pour son utilisation thérapeutique et/ou nutraceutique selon la présente invention dans laquelle la composition est administrée en une quantité permettant l'administration d'une dose d'huile de graine du fruit de *Punica granatum* par unité de poids de corps de 61 µg /kg.

La présente invention a également pour objet un dispositif d'administration comprenant la composition selon la présente invention.

Dans un mode de réalisation, le dispositif d'administration est un dispositif d'administration vétérinaire.

Dans un mode de réalisation, le dispositif d'administration est une pompe doseuse, une pipette, une seringue, un spray et/ou un patch.

La présente invention a également pour objet un kit comprenant d'une part un extrait de la peau du fruit de *Punica granatum,* sous forme particulaire ou sous forme d'extrait, telle que définie ci-dessus et d'autre part de l'huile de graine du fruit de *Punica granatum* telle que définie ci-dessus.

La figure 1 est une photographie des résultats des essais de dénaturation à la BSA tels que présentés en exemple 3, chaque tube correspondant respectivement de gauche à droite à la Sol. 1, Sol. 2, Sol. 3, Sol. 4.

La figure 2 est la représentation graphique des résultats des mesures d'absorbance mesurée à 660 nm des essais de dénaturation à la BSA tels que présentés en exemple 3, avec en ordonnée la valeur d'absorbance mesurée à 660 nm et en abscisse, respectivement de gauche à droite, la Sol. 1, Sol. 2, Sol. 3, Sol. 4.

Dans les exemples, tous les pourcentages sont donnés en volume, sauf indication contraire, la température est exprimée en degré Celsius sauf indication contraire, et la pression est la pression atmosphérique, sauf indication contraire.

### EXEMPLES

### Exemple 1 : Procédé de préparation de brovat de peau du fruit de Punica granatum.

### Exemple 1.1 : Procédé de préparation de brovat de peau du fruit de Punica granatum lyophilisée.

Les fruits de *Punica granatum* (variété Emec, Shani et Acco, origine : France) sont désinfectés par immersion dans de l'acide acétique. Ils sont ensuite pelés manuellement de manière à ne conserver que la peau.

Une quantité de 600 grammes de peaux obtenus est transférés dans un lyophilisateur (MARTIN CHRIST - Alpha 1-2 Plus), où une étape de congélation suivie d'une étape de lyophilisation sont effectuées selon les modalités suivantes :
La première étape de congélation s'effectue à une température de -20°C ;
Une étape de lyophilisation primaire s'effectue par application d'un vide à environ 60 micro-bars pendant 48h.

Les peaux de fruits lyophilisées sont broyées à l'aide d'un broyeur (MILIAN, Pulverisette 11) pendant 2 à 10min à une vitesse 2000 à 3000 tr/min.

De la peau de fruit lyophilisée et broyée est obtenue.

Une partie des peaux de fruits lyophilisées et broyées est remisée.

La teneur en polyphénols totaux est évaluée par dosage UV-VIS et selon la méthode décrite dans l'article Georgé S, et al. J Agric Food Chem. 2005 Mar 9;53(5): 1370-3.

La teneur en punicalagine et en acide ellagique est évaluée par HPLC selon la méthode décrite dans l'article Calín-Sánchez, Á., et al. Food Bioprocess Technol 6, 1644-1654 (2013) pour l'acide ellagique et la punicalagine

Les résultats sont présentés dans le tableau 1.

**Tableau 1**

| Substance active | Pourcentage massique (m/m) pour 100 grammes de peau de fruit lyophilisée et broyée est obtenue selon l'exemple 1 |
|---|---|
| Acide ellagique | 4.544 |
| Punicalagine | 28.320 |
| Polyphénols totaux | 33.23 |

### Exemple 1.2 : Procédé de préparation de l'extrait aqueux de broyât de peau du fruit de Punica granatum.

Une quantité de 5 g de broyat de peau lyophilisée tel qu'obtenu à l'exemple 1.1 est introduite dans un erlenmeyer. De l'eau chaude purifiée est versée sur la poudre pour atteindre un volume final de 200 ml, et la suspension obtenue est mélangée à l'aide d'une spatule une dizaine de fois.

Le mélange obtenu est laissé à refroidir jusqu'à atteindre la température ambiante, puis l'erlenmeyer est fermé et laissé reposer pendant 12h à une température de 4°C.

Le surnageant est filtré à l'aide d'un filtre à seringue et l'extrait aqueux de peau de fruit lyophilisées et broyées est conservé dans un flacon en verre scellé à 4°C.

### Exemple 2 : Procédé de préparation de la composition selon la présente invention.

### Exemple 2.1 : Procédé de préparation de la composition selon la présente invention comprenant un mélange sous forme d'émulsion huile dans eau.

L'huile de graine du fruit de *Punica granatum* (Origine : France) sous forme huile obtenue par séparation des graines des arilles les entourant, séchage et pressage à froid des graines, est achetée dans le commerce (Huile de pépin de grenade - L'Atelier d'Hippolyte). L'huile de graine du fruit de *Punica granatum* possède une teneur en acide punicique d'environ 70 g pour 100 g d'huile de graine du fruit de *Punica granatum.*

Une quantité de 200 µl d'huile de graine du fruit de *Punica granatum* est mélangée dans un mélangeur a haut-cisaillement à 2ml d'extrait aqueux de peau de fruit de *Punica granatum* tel qu'obtenu selon l'exemple 1.2. une quantité 2 % d'agent tensioactif est ajouté.

Une composition selon l'invention comprenant un mélange sous forme d'une émulsion huile dans eau est obtenue.

### Exemple 2.2 : Procédé de préparation de la composition selon la présente invention comprenant un mélange sous forme de poudre de peau de fruit de Punica granatum imprégnée d'huile de graine de Punica granatum.

Une quantité de 100 µl d'huile de graine du fruit de *Punica granatum* (Huile de pépin de grenade - L'Atelier d'Hippolyte) est mélangée sous agitation continue à 1000 mg de broyat de peau du fruit de *Punica granatum* lyophilisée tel qu'obtenu selon l'exemple 1.1.

Une composition selon l'invention comprenant un mélange sous forme d'une huile adsorbé sur une poudre de peau imprégnée est obtenue.

### Exemple 2.3. Procédé de préparation de la composition selon la présente invention comprenant un mélange sous forme de poudre.

Une quantité de 100 µl d'huile de graine du fruit de *Punica granatum* (Huile de pépin de grenade - L'Atelier d'Hippolyte) est mélangée sous agitation continue à 1 g de broyat de peau du fruit de *Punica granatum* lyophilisée tel qu'obtenu selon l'exemple 1.1. et de 500 mg de SYLOID^{®} (GRACE).

Une composition selon l'invention comprenant un mélange sous forme d'une poudre imprégnée est obtenue.

### Exemple 2.4. Procédé de préparation de la composition selon la présente invention sous forme semi solide.

Une phase aqueuse est préparée en mélangeant 0,05 g de cyclodextrine, 0,15 g de sirop de sorbitol et de l'eau QSP 100 mL.

Une phase huileuse est préparée en parallèle en mélangeant 10 mg de mélange sous forme de poudre tel que présenté à l'exemple 2.2 dans une huile MCT QSP 100 mL.

La phase huileuse est progressivement ajoutée dans la phase aqueuse pendant environ 30 minutes puis mélangé dans un mélangeur a haut-cisaillement.

Une composition sous la forme d'une émulsion huile dans eau selon l'invention est obtenue.

La composition obtenue est versée dans un dispositif de délivrance de spécialité liquide comportant un réservoir en gélatine mole et un capuchon fermant hermétiquement le réservoir, comportant en outre une languette disposée du côté opposé du capuchon par rapport au réservoir, la languette étant remplie de gélatine mole tel que décrit dans la demande WO2021156557 (A1).

### Exemple 3 : Démonstration de l'effet

### Exemple 3.1 : Démonstration de l'effet synergique de la combinaison selon l'invention.

Les tests de stabilisation de l'albumine sérique bovine (BSA, Carl Roth) pour évaluer le potentiel anti-inflammatoire et antiarthritique de nouveaux médicaments candidats ou d'extraits naturels est classiquement utilisé dans le domaine.

Les protéines dénaturées induisent la synthèse d'autoantigènes et la réponse immunitaire qui en résulte. En outre, l'albumine est une protéine de transport circulant naturellement dans le sang qui a la capacité de lier une grande variété de molécules, à la fois hydrosolubles et liposolubles, ce qui permet de tester un large spectre de composés.

Dans le présent test, le DMSO est utilisé en tant qu'agent dénaturant.

Les conditions expérimentales du présent essai imposant l'utilisation de solution, et non de particules, l'extrait aqueux de broyat de peau du fruit de *Punica granatum* préparé selon le protocole décrit à l'exemple 1.2 est utilisé. L'interprétation des résultats du présent exemple sont transposables à la peau du fruit de *Punica granatum* sous forme particulaire.

Une première étape de pré-incubation à 37°C pendant au moins 15 minutes de 10 ml de solution saline isotonique de BSA 5% (v/v) est réalisée.

Quatre solutions d'incubation sont préparées dans le DMSO et les volumes indiqués sont ajoutées aux 10 ml préparés ci-dessus :
Sol. 1 (contrôle) : 100µL de DMSO pur.
Sol. 2 : 100µL de solution comprenant 20% (v/v) de peau du fruit de *Punica granatum* préparé selon le protocole décrit à l'exemple 1.2 pour un volume final de la solution 2et du DMSO QSP.
Sol. 3 : 100µL de solution comprenant 2% (v/v) d'huile de graine du fruit de *Punica granatum* telle que décrite à l'exemple 2 pour un volume final de la solution 3 et du DMSO QSP.
Sol. 4 : 100µL de solution du composé comprenant 20% (v/v) d'extrait peau du fruit de *Punica granatum* préparé selon le protocole décrit à l'exemple 1.2, 2% (v/v) d'huile de graine du fruit de *Punica granatum* telle que décrite à l'exemple 2, et du DMSO QSP.

La Sol. 4 représente donc la condition correspondante à la composition selon la présente invention.

La différence de concentration en DMSO entre la condition 1 et les conditions 2,3 et 4 est négligeable pour l'interprétation des résultats.

Les solutions sont incubées à 37°C pendant 1 heure et passées au vortex toutes les 10 minutes. Les solutions sont ensuite réservées pendant 5 jours.

Des tubes à essais sont préparés avec 1960 µL de solution saline isotonique de BSA de manière d'obtenir une concentration finale de BSA de 0,1% (v/v), 750 µL de DMSO et 40 µL de l'une des solutions d'incubation. Les tubes ainsi préparés sont immergés dans un bain-marie à 70±2°C pendant 5 min, puis laissés à refroidir à température ambiante pendant 30min.

### Analyse spectrophotométrique :

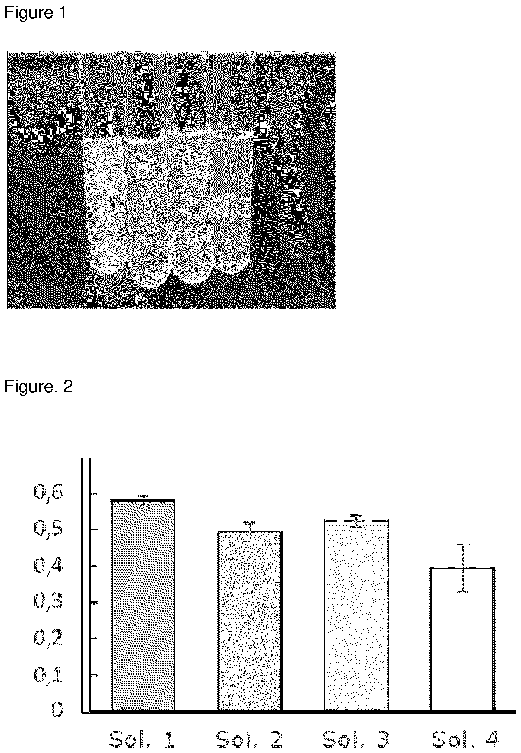

L'image présentée en figure 1 montre la différence observée visuellement après 5 minutes d'exposition de l'échantillon à 70±2°C entre le contrôle (seulement BSA, tube à gauche de l'image) et les échantillons testés (BSA + composé actif).

L'absorbance est mesurée à l'aide d'un spectrophotomètre (Heλios spectrophotometer) à 660nm. Les résultats d'absorbance alors obtenus sont présentés à la figure 2.

Le traitement de la BSA avec la Sol. 2 (peau du fruit de *Punica granatum*) permet de réduire la dénaturation de la protéine de 15% par rapport au contrôle.

Le traitement de la BSA avec le Sol.3 (huile de graine du fruit de *Punica granatum*) permet de réduire la dénaturation des protéines de 10% par rapport au contrôle.

De manière tout à fait surprenante, le traitement de la BSA avec la Sol. 4 (mélange peau et huile de graine du fruit de *Punica granatum*) conduit à une réduction de la dénaturation des protéines de 32 % par rapport au contrôle, soit une réduction supérieure à la somme des réductions deux composés pris individuellement.

Ainsi, cette étude démontre l'effet synergique du mélange de peau et d'huile de graine du fruit de *Punica granatum* pour protéger la protéine BSA contre la dénaturation.

Ces résultats démontrent donc un effet de synergie du mélange de peau du fruit de *Punica granatum* et d'huile de graine du fruit de *Punica granatum* pour le traitement des troubles articulaires, osseux, cartilagineux, inflammatoires, cardiovasculaires et/ou cognitifs.

### Exemple 3.2: Démonstration de l'effet synergique de la combinaison selon l'invention.

Le protocole selon l'exemple 3.1 a été répété avec différentes valeurs de teneur en peau du fruit de *Punica granatum* et d'huile de graine du fruit de *Punica granatum* telles que définies dans le tableau 2.

**Tableau 2**

| Solutions test | | Abs 660nm | % de protection |
|---|---|---|---|
| Sol. 1 | Contrôle | 0,565 | |
| Sol. 2 | Peau du fruit de *Punica granatum* 20% (v/v) | 0,530 | 6% |
| Sol. 3 | Peau du fruit de *Punica granatum* 40% (v/v) | 0,511 | 10% |
| Sol. 4 | Peau du fruit de *Punica granatum* 80% (v/v) | 0,495 | 12% |
| Sol. 5 | Huile de graine du fruit de *Punica granatum* 2% (v/v) | 0,520 | 8% |
| Sol. 6 | Huile de graine du fruit de *Punica granatum* 6,7% (v/v) | 0,510 | 10% |
| Sol. 7 | Huile de graine du fruit de *Punica granatum* 25% (v/v) | 0,448 | 21% |
| Sol. 8 | Peau du fruit de *Punica granatum* 20% + huile de graine du fruit de *Punica granatum* 2% | 0,420 | 26% |

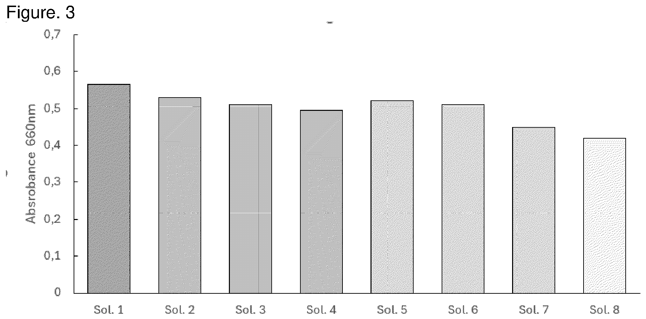

Les résultats sont également illustrés à la figure 3.

Ces résultats confirment l'effet synergique du mélange de peau et d'huile de graine du fruit de *Punica granatum* pour protéger la protéine BSA contre la dénaturation. Ces résultats confirment donc un effet de synergie du mélange de peau du fruit de *Punica granatum* et d'huile de graine du fruit de *Punica granatum* pour le traitement des troubles articulaires, osseux, cartilagineux, inflammatoires, cardiovasculaires et/ou cognitifs.

## Revendications

1. Composition comprenant de la peau du fruit de *Punica granatum,* sous forme particulaire ou sous forme d'extrait, et de l'huile de graine du fruit de *Punica granatum* en mélange, dans laquelle le ratio entre la teneur massique de peau du fruit de Punica granatum, sous forme particulaire ou sous forme d'extrait, et la teneur massique d'huile de graine du fruit de Punica granatum est compris dans un intervalle allant de 7/1 à 15/1.

2. Composition selon la revendication 1, dans laquelle la peau du fruit de *Punica granatum* sous forme particulaire est un broyat de peau du fruit de *Punica granatum.*

3. Composition selon l'une quelconque des revendications 1 ou 2, dans laquelle la peau du fruit de *Punica granatum* sous forme d'extrait est un extrait aqueux de peau du fruit de *Punica granatum.*

4. Composition selon l'une quelconque des revendications précédentes, dans laquelle la peau du fruit de *Punica granatum* est lyophilisée.

5. Composition selon l'une quelconque des revendications précédentes, dans laquelle l'huile de graine du fruit de *Punica granatum* est obtenue par pressage à froid.

6. Composition selon l'une quelconque des revendications précédentes, le ratio entre la teneur massique de peau du fruit de Punica granatum, sous forme particulaire ou sous forme d'extrait, et la teneur massique d'huile de graine du fruit de Punica granatum est compris dans un intervalle allant de 9/1 à 12/1.

7. Composition selon l'une quelconque des revendications précédentes, dans laquelle la composition est une composition cosmétique, nutraceutique et/ou pharmaceutique et/ou un ingrédient alimentaire et/ou un complément alimentaire et/ou actif cosmétique, nutraceutique et/ou pharmaceutique.

8. Composition selon l'une quelconque des revendications précédentes, dans laquelle la composition selon la présente invention comprend en outre au moins un excipient pharmaceutiquement et/ou cosmétiquement acceptable.

9. Composition selon l'une quelconque des revendications 1 à 8 pour son utilisation thérapeutique et/ou nutraceutique.

10. Composition pour son utilisation selon la revendication 9, pour le traitement et/ou la prévention des troubles articulaires, osseux, cartilagineux, inflammatoires, cardiovasculaires et/ou cognitifs.

11. Composition pour son utilisation selon l'une quelconque des revendications 9 ou 10, pour le traitement et/ou la prévention des troubles articulaires, osseux et/ou cartilagineux.

12. Composition pour son utilisation selon l'une quelconque des revendications 9 ou 10, pour le traitement et/ou la prévention de la dermatite atopique, de l'arthrose, de la polyarthrite rhumatoïde et/ou de l'ostéoporose et/ou pour maintenir et/ou améliorer le remodelage osseux.

13. Composition pour son utilisation selon l'une quelconque des revendications 10 ou 12, dans laquelle la composition est administrée chez l'animal.

14. Dispositif d'administration comprenant la composition selon l'une quelconque des revendications 1 à 8.

15. Dispositif d'administration selon la revendication 1 à 8, dans lequel le dispositif d'administration est un dispositif d'administration vétérinaire.
